# EUROPEAN PATENT SPECIFICATION

(11) **EP 2 903 586 B1**
(45) Date of publication and mention of the grant of the patent: **22.01.2020**
(21) Application number: 13811596.9
(22) Date of filing: 07.10.2013
(51) Int. Cl.: A61K 8/34, A61K 8/37, A61Q 7/02, A61K 8/22

(54) **COMPOSITION**
ZUSAMMENSETZUNG
COMPOSITION

(30) Priority: 05.10.2012 GB 201217915
(43) Date of publication of application: 12.08.2015
(73) Proprietor: Trigiante, Giuseppe, Albert Road South Watford WD17 1QQ (GB)
(72) Inventor: Trigiante, Giuseppe, Albert Road South Watford WD17 1QQ (GB)
(74) Representative: Wallace, Sheila Jane
(86) International application number: PCT/GB2013/052606
(87) International publication number: WO 2014/053860

(56) References cited:
- WO-A1-01/80816
- WO-A1-96/09853
- WO-A1-2014/055169
- DE-A1- 10 163 579
- FR-A1- 2 897 533
- US-A1- 2008 234 374
- "Butyl octanoate", FOOD AND CHEMICAL TOXICOLOGY, PERGAMON, GB, vol. 38, 1 January 2000 (2000-01-01), pages s23-s24, XP025950002, ISSN: 0278-6915 [retrieved on 2000-01-01]
- LOTH ET AL: "Vehicular influence on transdermal drug penetration", INTERNATIONAL JOURNAL OF PHARMACEUTICS, ELSEVIER BV, NL, vol. 68, no. 1-3, 1 February 1991 (1991-02-01), pages 1-10, XP025568395, ISSN: 0378-5173, DOI: 10.1016/0378-5173(91)90120-D [retrieved on 1991-02-01]
- RAWLINGS J M ET AL: "Effect of Dosing Vehicle on the Dermal Absorption of Fluazifop-butyl and Fomesafen in Rats in Vivo", FUNDAMENTAL AND APPLIED TOXICOLOGY, SOCIETY OF TOXICOLOGY, AKRON, OH, US, vol. 23, no. 1, 1 July 1994 (1994-07-01), pages 93-100, XP024875767, ISSN: 0272-0590, DOI: 10.1006/FAAT.1994.1084 [retrieved on 1994-07-01]

## Description

This invention relates to compositions for inhibiting the re-growth of hair from a vacant hair follicle, methods for preparing such compositions and the use of such compositions to inhibit the re-growth of hair and in the treatment of conditions characterised by excessive undesired hair growth, such as congenital hypertrichosis, acquired hypertrichosis and hirsutism, as well as kits comprising containers containing components of the composition. The invention also provides methods for removing hair and inhibiting its re-growth.

The overabundance of body hair (hirsutism) is caused by many factors, mainly genetic and hormonal, but it can also be caused by chemicals and medicines, such as minoxidil (see Erkkola R, "Hirsutism: definitions and etiology" Ann. Med., 22(2):99-103 (1990)). The male hormone testosterone plays a pivotal role in hormonal hirsutism as its effect on body hair is to thicken and pigment the hair. Paradoxically, the same hormone causes an opposite effect on scalp hair, provoking androgenetic alopecia (male pattern baldness), and the two phenotypes are commonly associated. This effect is not visible before puberty and, though seen mostly in males, also affects females. Excessive body hair is often seen as undesirable and its presence can cause distress in affected people. There is therefore a growing market pressure for methods to remove it.

Hair removal has a very long history (see Scott et al., "Epilation," Cutis, 46(3), 216- 217 (1990)). In ancient times all sorts of fanciful methods were used for the purpose, including tweezing and shaving with shells and pumice stones, sugaring, waxing and dissolution of the hair with caustic substances. Most of those treatments were painful and unsafe for the skin. Moreover, they caused no permanent disappearance of the hair which instead grew back after varying periods. Achieving permanent hair removal is much more difficult and it is only recently that permanent hair removal methods have been developed.

Hair removal by chemical means is an established procedure but the effect is always temporary as no currently available chemical formulation is capable of penetrating deep into the hair follicle and reaching the hair regenerative compartment, called the dermal papilla. Therefore, efficacy is limited to the hair shaft itself which is quickly regenerated by the papilla. This is because of the hair follicle structure. Every hair follicle comprises a dark, elongating column of cornified dead cells called the hair shaft. This is the visible hair and extends from the bulb to above the skin. At the base of the shaft lies a proliferating bulb which consists of a matrix of actively proliferating keratinocytes, which produce the hair shaft, surrounding a pine cone shaped organ made of mesenchimal (dermal) cells called the dermal papilla. This organ centralizes control of hair growth. It provides the matrix with the chemical signals to proliferate, thus causing hair growth. At the same time it governs the hair growth cycle, which is typical of all hair.

Every hair undergoes a growth phase called anagen, followed by a shedding phase called telogen, at the end of which the hair falls out and a new matrix is summoned by the dermal papilla to produce a new shaft (see Bowden, et al., "Characterization and chromosomal localization of human hair-specific keratin genes and comparative expression during the hair growth cycle," J. Invest Dermatol., 110(2), 158-164 (1998)). This cycle continues throughout life and it is not influenced by shaving, because the external portion of the hair shaft is made of dead tissue and cannot communicate any information to the bulb. Every time a hair is plucked the traumatic event causes loss of the whole matrix and hair shaft but the dermal papilla is almost always left behind; if this was not the case, tweezing would be permanent. The dermal papilla then restarts hair production just as if the hair had fallen out on its own without human intervention. This is why all hair removal methods that act on the hair shaft are temporary. Permanent hair removal can only be achieved by inactivating the dermal papilla.

Current hair removal methods can be classified in two categories: methods which target the hair shaft (*e.g*., shaving, tweezing, waxing, sugaring) and methods which try to target the hair dermal papilla (*e.g*., electrolysis and laser treatment). The former are easy to implement but can only provide temporary effect. Targeting the dermal papilla is a much more complicated task because it lies deep in the dermis and it cannot be removed by mechanical means. It is very difficult to damage it without causing skin damage at the same time. Only recently have two technologies come about that are able to target this organelle effectively.

Hair electrolysis was first described in 1875 by a physician called Charles E. Michels. The technique involves inserting a fine metal conductor down into the hair shaft. An electric current is then applied and the hair bulb is destroyed either by overheating (thermal electrolysis) or by the electrochemical local generation of caustic compounds (galvanic electrolysis). Because the hair bulb is targeted, the technique provides permanent hair removal. The main drawbacks of electrolysis are its slow rate (only one follicle can be treated per application), its painfulness, and the risk of scarring if sufficient care is not exerted. Nevertheless, electrolysis is now very popular throughout the world.

The most modern method available for permanent hair removal is performed using a laser (see Mandt et al., "Epilation today: Physiology of the hair follicle and clinical photo- epilation," J. Investig. Dermatol. Symp. Proc., 10(3), 271-274 (2005)). This technique was first discovered accidentally in the late 1970s and has since developed into the most popular technology on the market. Laser hair removal works by applying intense pulses of laser light at a certain wavelength on the hair after it has been shaven off. The principle is that, as it is a different colour to the rest of the skin, the hair shaft will absorb more of the laser radiation, which causes the shaft to overheat. The heat energy produced is transmitted down the shaft and into the dermal papilla, thereby damaging it.

This method is effective, although not perfect, and it does reduce the coarseness of most hair. However, it also comes with drawbacks. Because of its principle of action, it works best where there is a strong colour contrast between the hair and surrounding skin, *i.e.* ideally black hair on white skin. When this is not the case, such as for light hair or dark complexions, the method is rather less effective and can cause skin damage. Indeed, it can instead often cause irritation. It is also expensive because of the equipment involved and must be carried out in specialized salons. Finally, because of its unpredictable efficiency, it cannot be considered a "permanent hair removal" method. Rather, it should be considered as a "permanent hair reduction" method and it is so advertised.

Chemical depilatory products are also available and these are generally in the form of creams, lotions and ointments. Well known examples include products sold under the trade names Veet® and Nair®. These products are readily available over-the-counter and offer a fast, pain-free and relatively inexpensive way of removing hair. However, unlike laser and electrolysis hair removal procedures, the effects are not permanent and re-growth is usually observed within 2 to 5 days. In this context, the document WO9609853 is relevant.

In view of the above, there is an obvious need for the development of a relatively inexpensive, pain-free, straightforward and effective method for achieving the permanent removal of hair. Furthermore, it is desirable that such a treatment is available "over-the-counter" so that hair removal can be performed by an individual in the comfort of their own home without the need to visit a healthcare professional.

The present applicant has previously disclosed compositions for hair removal comprising an organic hair follicle penetrating agent and an enzyme inhibitor of cellular metabolism (WO 2008/117122). Although these compositions could permanently prevent the re-growth of hair following epilation, the toxic nature of the cellular metabolism inhibitors meant that the compositions were not suitable for over-the-counter sale and required application by trained healthcare professionals. Therefore, there is a need to develop an effective permanent hair removal composition suitable for use by an individual in the comfort of their own home.

In view of the above, the applicant has developed a composition endowed with the ability to reach the dermal papilla of a vacant hair follicle and inactivate it, therefore inhibiting re-growth and ultimately resulting in permanent inactivation of the hair follicle that is suitable for home use and does not require administration by a healthcare professional.

According to the present invention, there is provided a composition for inhibiting the re-growth of hair from a vacant hair follicle comprising at least one organic hair follicle penetrating agent and at least one oxidising agent, characterised in that the at least one organic hair follicle penetrating agent is a C₇ to C₁₆ ester or a mixture thereof and that the total weight of the hair follicle penetrating agent is from 50% to 99% by weight of the composition, wherein the at least one oxidising agent is selected from a peroxide based oxidising agent or a chlorine based oxidising agent, and wherein the peroxide based oxidising agent is hydrogen peroxide, *tert-butyl* hydroperoxide, benzoyl peroxide, cumene hydroperoxide, peroxydisulfuric acid or a salt thereof, peroxyphosphoric acid or a salt thereof, peroxyacetic acid or a salt thereof, peroxypropionic acid or a salt thereof, or peroxybutyric acid or a salt thereof.

For the avoidance of doubt, when used herein, any ranges presented are inclusive of the end-points.

When used herein, the term *"vacant hair follicle"* refers to a hair follicle that has had the hair previously contained within it removed by epilation. The vacant hair follicle still contains a dermal papilla.

When used herein, the term *"hair follicle penetrating agent"* refers to any organic compound that can enter the narrow openings of the hair follicle (which are approximately tens of microns in diameter) and penetrate the entirety of the follicle cavity.

In certain embodiments of the present invention the oxidising agent is a peroxide based oxidising agent.

When used herein, the term *"peroxide based oxidising agent"* refers to an oxidising agent that contains a peroxide moiety, that is, an oxygen-oxygen single bond.

The peroxide based oxidising agent is hydrogen peroxide, *tert*-butyl hydroperoxide, benzoyl peroxide, cumene hydroperoxide, peroxydisulfuric acid or a salt thereof, peroxyphosphoric acid or a salt thereof, peroxyacetic acid or a salt thereof, peroxypropionic acid or a salt thereof, or peroxybutyric acid or a salt thereof. More preferably, the peroxide based oxidising agent is hydrogen peroxide.

In other embodiments of the present invention, the oxidising agent is a chlorine based oxidising agent.

When used herein, the term *"chlorine based oxidising agent"* refers to an oxidising agent that contains a chlorine atom bonded to a heteroatom.

Preferably, the chlorine based oxidising agent is sodium hypochlorite, calcium hypochlorite, trichloroisocyanuric acid or a salt thereof (*e.g*. sodium trichloroisocyanurate), dichloroisocyanuric acid or a salt thereof, monochloroisocyanuric acid or a salt thereof, an inorganic chloramine, chloramine T, or halozone. More preferably, the chlorine based oxidising agent is trichloroisocyanuric acid or a salt thereof.

The at least one organic hair follicle penetrating agent is a C₇ to C₁₆ ester or a mixture thereof.

When discussed herein, esters are referred to as "*Cₓ*" esters, wherein X indicates the total number of carbon atoms in the ester molecule.

In a preferred embodiment, the ester is a C₈ ester, such as heptyl methanoate; hexyl ethanoate; pentyl propanoate; butyl butanoate; propyl pentanoate; ethyl hexanoate; and methyl heptanoate (*e.g*. butyl butanoate).

In a further preferred embodiment, the ester is a C₁₂ ester, such as undecyl methanoate; decyl ethanoate; nonyl propanoate; octyl butanoate; heptyl pentanoate; hexyl hexanoate; pentyl heptanoate; butyl octanoate; propyl nonanoate; ethyl decanoate; and methyl undecanoate (e.g. hexyl hexanoate).

In certain embodiments of the present invention, the total weight of the oxidising agent is from 0.1% to 30% by weight of the composition, preferably from 0.1% to 20% by weight, more preferably from 0.1% to 10% by weight, even more preferably from 1% to 10% by weight, particularly from 1% to 7%, and especially from 2% to 6% by weight. Ranges such as from 2.5% to 3.5% by weight, *e.g*. approximately 3%, or from 4.5% to 5.5% by weight, *e.g*. approximately 5%, are particularly preferred.

In embodiments of the present invention wherein a peroxide based oxidising agent is used, the total weight of the oxidising agent may be from 0.1% to 20% by weight of the composition, preferably from 0.1% to 10% by weight, more preferably from 1% to 10% by weight, particularly 1% to 6% by weight, and especially from 2.5% to 3.5% by weight, *e.g*. approximately 3%.

In embodiments of the present invention wherein a chlorine based oxidising agent is used, the total weight of the oxidising agent may be from 0.1% to 20% by weight of the composition, preferably from 0.1% to 10% by weight, more preferably from 1% to 10% by weight, particularly from 1% to 7% by weight and especially from 3% to 7% by weight, *e.g*. approximately 5%.

The total weight of the organic hair follicle penetrating agent is from 50% to 99% by weight of the composition.

In other embodiments of the present invention, the composition further comprises a solvent such as a polar protic solvent or a polar aprotic solvent.

Preferably, the polar protic solvent is an alcohol such as ethanol, propanol or isopropanol (*e.g*. ethanol).

Preferably, the polar aprotic solvent is a C3 to C6 ester or ketone , or a mixture thereof. Suitable examples include acetone, butanone, pentanone, ethyl ethanoate, propyl ethanoate, ethyl propanoate and propyl propanoate.

When a peroxide based oxidising agent is used, the solvent can be a protic solvent or an aprotic solvent. However, when the oxidising agent is a chlorine based oxidising agent, an aprotic solvent should be used

In certain embodiments the total weight of the solvent is from 0.1% to 50% by weight of the composition, preferably from 5% to 50% by weight, more preferably from 10% to 40% by weight, particularly from 20% to 40% by weight, and especially from 25% to 35% by weight (*e.g*. approximately 28%).

In certain embodiments of the invention the composition comprises a peroxide based oxidising agent in an amount from 1% to 5% (*e.g*. approximately 3%) by weight of the composition; an organic hair follicle penetrating agent in an amount from 60% to 70% (*e.g*. approximately 65%) by weight of the composition; and a solvent in an amount from 27% to 37% (*e.g*. approximately 32%) by weight of the composition.

In other specific embodiments, the composition comprises hydrogen peroxide in an amount from 1% to 5% by weight of the composition (preferably from 2.5% to 3.5% by weight *e.g*. approximately 3%); a C₈ ester (*e*.*g*. butyl butanoate) in an amount from 50% to 80% by weight, preferably from 50% to 70% by weight (especially from 60% to 70% by weight, *e.g.* approximately 65%); an alcohol (*e.g*. ethanol) in an amount from 1% to 50% by weight, preferably from 10% to 40% by weight, more preferably from 20% to 40% by weight, and particularly from 25% to 35% by weight (especially from 27% to 29% by weight, *e.g*. approximately 28%); and water in an amount from 1% to 10% by weight (preferably from 4% to 6% by weight, *e.g*. approximately 5%).

In further specific embodiments, the composition comprises hydrogen peroxide in an amount from 1% to 5% by weight of the composition (preferably from 2.5% to 3.5% by weight *e.g*. approximately 3%); a C₁₂ ester (*e.g.* hexyl hexanoate) in an amount from 50% to 80% by weight, preferably from 50% to 70% by weight (especially from 60% to 70% by weight, *e.g*. approximately 65%); an alcohol (*e.g*. ethanol) in an amount from 1% to 50% by weight, preferably from 10% to 40% by weight, more preferably from 20% to 40% by weight, and particularly from 25% to 35% by weight (especially from 27% to 29% by weight, *e.g*. approximately 28%); and water in an amount from 1% to 10% by weight (preferably from 4% to 6% by weight, *e.g*. approximately 5%).

In other specific embodiments, the composition comprises: trichloroisocyanuric acid in an amount from 1% to 10% by weight of the composition, preferably from 1% to 7% or 4% to 6% by weight (*e.g*. approximately 5%); and a C₈ or C₁₂ ester (*e.g*. butyl butanoate or hexyl hexanoate) in an amount from 50% to 99% by weight, preferably from 70% to 99% by weight, more preferably from 80% to 99% by weight, particularly from 90% to 99% by weight, and especially from 93% to 96% by weight (*e.g*. approximately 94% to 95%).

In further specific embodiments, the composition comprises: trichloroisocyanuric acid in an amount from 1% to 10% by weight of the composition, preferably from 1% to 7% or 3% to 6% by weight (*e.g*. approximately 5%); a C₈ or C₁₂ ester (*e.g*. butyl butanoate or hexyl hexanoate) in an amount from 50% to 99% by weight, preferably from 70% to 95% by weight, more preferably from 80% to 90% by weight, (*e.g*. approximately 84% to 88%); and a C₃ or C₄ ketone or ester (e.g. acetone or ethyl ethanoate) in an amount from 0.1% to 45% by weight, preferably from 0.1% to 25% by weight, more preferably from 5 to 15% by weight (e.g. approximately 9%) .

According to the present invention there is also provided a method for preparing a composition according to the present invention comprising forming a mixture of an oxidising agent as described above and an organic hair follicle penetrating agent as described above, and optionally a solvent and/or one or more additional excipients, wherein the concentration of the oxidising agent in the resultant composition is between 0.1% and 10% by weight of the composition.

In another aspect, the present invention provides a method for removing hair and inhibiting its re-growth comprising the steps of removing hair from an area of skin by epilation; and topically applying a composition according to the present invention as described above onto the area for a period of time (preferably from one to ten minutes *e.g.* 5 minutes) sufficient to inhibit the re-growth of hair from the vacant follicles.

In certain embodiments, the composition may be removed from the application area after a specified period of time (preferably from one to ten minutes *e.g*. 5 minutes) has elapsed.

The composition is applied to an area of skin which is to be treated prior to epilation.

Another aspect of the present invention provides a composition according to the invention for use in the treatment of a condition characterised by excessive undesired hair growth, such as congenital hypertrichosis, acquired hypertrichosis and hirsutism.

Another aspect of the present invention includes the use of a composition according to the invention to inhibit the re-growth of hair from a vacant hair follicle.

A further aspect of the present invention is a kit comprising a first container that contains an oxidising agent as described above; and a second container that contains an organic hair follicle penetrating agent as described above. The kit may also contain instructions for use of the components comprising the kit. For instance, the instructions may provide directions for mixing the contents of the first and second containers prior to application or for the sequential application of the contents of the first and second containers. Methods of application may also be described. The instructions may also specify the length of time that the components of the kit should be applied to an area of skin, either sequentially or together, and/or provide instructions for removal of the components or the resultant composition from the application area.

In a further aspect the present invention provides a kit comprising a container containing a composition according to the present invention. Such a kit may also contain instructions for use of the composition in the container. For instance, the instructions may provide directions for the application of the composition. The instructions may also specify the length of time that the composition should be applied to an area of skin and/or provide instructions for removal of the composition from the application area.

In another aspect of the present invention, there is provided a kit comprising a first container that includes an oxidising agent and a second container that includes an organic hair follicle penetrating agent. The kit may optionally include a third container including a solvent or a mixture of solvents for the purpose of rendering the oxidising agent and hair follicle penetrating agent miscible.

In embodiments of these aspects, the kit may further include a device to topically administer a composition according to the present invention to the site of administration.

### Detailed Description of the Invention

The environment near the hair follicle is a highly hydrophobic environment which contains sebum from sebaceous glands and fatty secretions from the skin. The highly hydrophobic environment prevents any watery or water based solution from penetrating the hair follicle, because water has a high surface tension which prevents its surface from assuming the high curvature necessary to enter narrow openings such as those of a hair follicle. Besides, the hydrophobicity of the local environment does not allow water based solutions to wet the surface of the skin. The main challenge is therefore to devise a suitable penetration formula such as that presented by the composition of the present invention.

The invention encompasses a different approach to permanent hair removal than water based topical compositions. Not wishing to be bound by theory, it is believed that the present invention is based on the fact that, following removal of hair such as by epilation (plucking), the hair follicle is temporarily left vacant and therefore potentially accessible to a formulation able to penetrate said follicle. An appropriate composition could deliver any suitable cytotoxic agent to the dermal papilla without penetrating into and therefore damaging the surrounding skin. Hence, when the agent is a cytotoxic compound, then the dermal papilla could be inactivated and subsequent hair growth prevented. However, an obstacle to this approach is the extremely minute diameter of the hair follicle, which can be in the order of 30 micron (30 µm) (see Bowden et al., "Characterization and chromosomal localization of human hair-specific keratin genes and comparative expression during the hair growth cycle," J. Invest. Dermatol., 110 (2), 158-164 (1998)).

A first aspect of the present invention provides topical compositions for inhibiting hair re-growth from a vacant follicle, designed to be applied to the skin after hair has been removed from its follicle by epilation and ideally, the composition should be applied to the skin before hair has begun to regenerate. When used herein, the term *"epilation"* refers to the process of removal of an entire hair from its hair follicle. Epilation may be achieved by any known method, such as waxing, threading, sugaring, tweezing etc. Once applied to the skin, it is postulated that the compositions according to the present invention penetrate into the hair follicle and deliver the active cytotoxic agent, in this case an oxidising agent, to the dermal papilla. The oxidising agent causes damage to the hair bulb and cells of the dermal papilla such that the hair follicle is permanently inactivated and re-growth of hair is prevented.

According to the present invention, the topical composition comprises at least one organic hair follicle penetrating agent and at least one oxidising agent, wherein the at least one oxidising agent is selected from a peroxide based oxidising agent or a chlorine based oxidising agent, and wherein the peroxide based oxidising agent is hydrogen peroxide, *tert*-butyl hydroperoxide, benzoyl peroxide, cumene hydroperoxide, peroxydisulfuric acid or a salt thereof, peroxyphosphoric acid or a salt thereof, peroxyacetic acid or a salt thereof, peroxypropionic acid or a salt thereof, or peroxybutyric acid or a salt thereof. The organic hair follicle penetrating agent is a C₇ to C₁₆ ester, or a mixture thereof and serves to deliver the oxidising agent to a vacant follicle so as to provoke cellular death inside the follicle lining (*i.e.* the dermal papilla).

In certain embodiments of the present invention, the oxidising agent may be present in an amount from 0.1% to 30% by weight of the composition, preferably 0.1% to 20% by weight, more preferably from 0.1% to 10% by weight, more preferably from 1% to 10% by weight, even more preferably from 1% to 7% by weight, and preferably from 2% to 6% by weight (especially from 2.5% to 3.5% by weight, *e.g*. approximately 3%, or from 4.5% to 5.5% by weight, *e.g*. approximately 5%).

In certain preferred embodiments, the oxidising agent is a peroxide based oxidising agent selected from hydrogen peroxide; *tert*-butyl hydroperoxide; benzoyl peroxide; cumene hydroperoxide; peroxydisulfuric acid or a salt thereof; peroxyphosphoric acid or a salt thereof; peroxyacetic acid or a salt thereof; peroxypropionoic acid or a salt thereof; or peroxybutyric acid or a salt thereof. Preferred peroxide based oxidising agents are hydrogen peroxide, *tert*-butyl hydroperoxide and benzoyl peroxide.

Preferably, hydrogen peroxide is used as the oxidising agent. Typically, aqueous hydrogen peroxide is used in the composition. Such aqueous solutions may contain hydrogen peroxide in an amount from 3% to 50% by weight of the solution. For example, the aqueous solutions may contain 3%, 30%-32%, or 35% hydrogen peroxide by weight. Preferably, the hydrogen peroxide solutions have been approved for use in either the cosmetic or food industries.

The aqueous hydrogen peroxide solutions may also comprise a suitable peroxide stabilizing agent and such agents are well known to those skilled in the art.

In embodiments of the present invention wherein the composition comprises a peroxide based oxidizing agent, said agent is typically present in an amount from 0.1% to 20% by weight of the composition, preferably from 0.1% to 10% by weight, more preferably from 1% to 10% by weight, even more preferably from 1% to 6% by weight, and most preferably from 2.5% to 3.5% by weight (*e.g*. approximately 3% by weight).

When hydrogen peroxide is the oxidising agent, it is typically present in an amount from 0.1% to 6% by weight of the composition, preferably from 1% to 6% by weight, more preferably from 2% to 4% by weight, even more preferably from 2.5% to 3.5% by weight. Most preferably, hydrogen peroxide is present in an amount of approximately 3% by weight of the composition.

In other preferred embodiments, the composition comprises a chlorine based oxidising agent. Examples of such an agent include a perchlorate (such as perchloric acid or a salt thereof, *e.g*. ammonium perchlorate, sodium perchlorate monohydrate or magnesium perchlorate); a chlorate (such as barium chlorate, calcium chlorate, strontium chlorate, zinc chlorate, potassium chlorate or sodium chlorate); a chlorite (such as sodium chlorite); a hypochlorite (such as lithium hypochlorite and calcium hypochlorite); monochloroisocyanuric acid or a salt thereof; dichloroisocyanuric acid or a salt thereof; trichloroisocyanuric acid or a salt thereof; an inorganic chloramine (such as monochloramine (NH₂Cl), dichloramine (NHCl₂) or trichloramine (NCl₃)); or an organic chloramine (such as *N*-chloromorpholine, *N*-chloropiperidine, *N-*chloroquinuclidinium chloride, sodium chloro[(4-methyl phenyl)sulfonyl]azanide (chloramine-T), or *p*-(*N*,*N*-Dichlorosulfamoyl)benzoic acid (halozone)).

In preferred embodiments, the chlorine based oxidising agent may be sodium hypochlorite, calcium hypochlorite, trichloroisocyanuric acid or a salt thereof, dichloroisocyanuric acid or a salt thereof, monochloroisocyanuric acid or a salt thereof, an inorganic chloramine, chloramine-T, or halozone. In the most preferred embodiments, the chlorine based oxidising agent is trichloroisocyanuric acid or a salt thereof (*e.g*. sodium trichloroisocyanurate).

In embodiments of the present invention wherein the composition comprises a chlorine based oxidizing agent, said agent is typically present in an amount from 0.1% to 20% by weight of the composition, preferably from 0.1% to 10% by weight, more preferably from 1% to 10% by weight, even more preferably from 1% to 7% by weight, and most preferably from 3% to 7% (*e.g*. approximately 5% by weight).

When trichloroisocyanuric acid or a salt thereof (*e.g*. sodium trichloroisocyanurate) is the oxidising agent, it is typically present in an amount from 0.1% to 7% by weight of the composition, preferably from 3% to 7% by weight, even more preferably from 4% to 6% by weight, and most preferably in an amount of approximately 5% by weight of the composition.

The composition of the present invention comprises at least one organic hair follicle penetrating agent. The follicle penetrating agent is an ester or a mixture thereof.

The organic hair follicle penetrating agent should be present in an amount sufficient to allow the oxidising agent to reach the hair follicle. The organic hair follicle penetrating agent is present in amount from 50% to 99% by weight.

In embodiments of the present invention the organic hair follicle penetrating agent is a C₇ to C₁₆ ester or a mixture thereof. In more preferred embodiments, the ester may be a C₇, C₈ or C₉ ester or a mixture thereof. In the most preferred embodiments, the ester is a C₈ or C₁₂ ester or a mixture thereof. Butyl butanoate is the most preferred C₈ ester, and hexyl hexanoate is the most preferred C₁₂ ester.

Examples of C₇ esters that may be used as follicle penetrating agents in embodiments of the invention include hexyl methanoate; pentyl ethanoate; butyl propanoate; propyl butanoate; ethyl pentanoate; and methyl hexanoate. The most preferred C₇ ester is propyl butanoate.

Examples of C₈ esters that may be used as follicle penetrating agents in embodiments of the invention include heptyl methanoate; hexyl ethanoate; pentyl propanoate; butyl butanoate; propyl pentanoate; ethyl hexanoate; and methyl heptanoate. In a certain preferred embodiment, the organic hair follicle penetrating agent is butyl butanoate, which can be obtained commercially from, for example, Sigma Aldrich (www.sigmaaldrich.com) in a purity of ≥98% as measured by gas chromatography.

Examples of C₉ esters that may be used as follicle penetrating agents in embodiments of the invention include octyl methanoate; heptyl ethanoate; hexyl propanoate; pentyl butanoate; butyl pentanoate; propyl hexanoate; ethyl heptanoate; and methyl octanoate. The most preferred C₉ esters are pentyl butanoate or butyl pentanoate.

Examples of C₁₀ esters that may be used as follicle penetrating agents in embodiments of the invention include nonyl methanoate; octyl ethanoate; heptyl propanoate; hexyl butanoate; pentyl pentanoate; butyl hexanoate; propyl heptanoate; ethyl octanoate; and methyl nonanoate. Preferably, the C₁₀ ester is pentyl pentanoate, butyl hexanoate or hexyl butanoate and most preferably butyl hexanoate or hexyl butanoate.

Examples of C₁₁ esters that may be used as follicle penetrating agents in embodiments of the invention include decyl methanoate; nonyl ethanoate; octyl propanoate; heptyl butanoate; hexyl pentanoate; pentyl hexanoate; butyl heptanoate; propyl octanoate; ethyl nonanoate; and methyl decanoate. Preferably, the C₁₁ ester is hexyl pentanoate or pentyl hexanoate.

Examples of C₁₂ esters that may be used as follicle penetrating agents in embodiments of the invention include undecyl methanoate; decyl ethanoate; nonyl propanoate; octyl butanoate; heptyl pentanoate; hexyl hexanoate; pentyl heptanoate; butyl octanoate; propyl nonanoate; ethyl decanoate; and methyl undecanoate. Preferably, the C₁₀ ester is heptyl pentanoate, hexyl hexanoate or pentyl heptanoate and most preferably heptyl pentanoate or pentyl heptanoate.

Examples of C₁₃ esters that may be used include dodecyl (lauryl) methanoate; undecyl ethanoate; decyl propanoate; nonyl butanoate; octyl pentanoate; heptyl hexanoate; hexyl heptanoate; pentyl octanoate; butyl nonanoate; propyl decanoate; ethyl undecanoate; and methyl dodecanoate (laurate). Preferably, the C₁₃ ester is heptyl hexanoate or hexyl heptanoate.

Examples of C₁₄ esters that may be used as follicle penetrating agents in embodiments of the invention include tridecyl methanoate; dodecyl (lauryl) ethanoate; undecyl propanoate; decyl butanoate; nonyl pentanoate; octyl hexanoate; heptyl heptanoate; hexyl octanoate; pentyl nonanoate; butyl decanoate; propyl undecanoate; ethyl dodecanoate (laurate); and methyl tridecanoate. Preferably, the C₁₄ ester is heptyl heptanoate, octyl hexanoate or hexyl octanoate and most preferably octyl hexanoate or hexyl octanoate.

Examples of C₁₅ esters that may be used as follicle penetrating agents in embodiments of the invention include tetradecyl (myristyl) methanoate; tridecyl ethanoate; dodecyl (lauryl) propanoate; undecyl butanoate; decyl pentanoate; nonyl hexanoate; octyl heptanoate; heptyl octanoate; hexyl nonanoate; pentyl decanoate; butyl undecanoate; propyl dodecanoate (laurate); ethyl tridecanoate; and methyl tetradecanoate (myristate). Preferably, the C₁₅ ester is octyl heptanoate or heptyl octanoate.

Examples of C₁₆ esters that may be used as follicle penetrating agents in embodiments of the invention include pentadecyl methanoate; tetradecyl ethanoate; tridecyl propanoate; dodecyl butanoate; undecyl pentanoate; decyl hexanoate; nonyl heptanoate; octyl octanoate; heptyl nonanoate; hexyl decanoate; pentyl undecanoate; butyl dodecanoate (laurate); propyl tridecanoate; ethyl tetradecanoate (myristate); and methyl pentadecanoate (myristate). Preferably, the C₁₆ ester is octyl octanoate, nonyl heptanoate or heptyl nonanoate and, most preferably, nonyl heptanoate or heptyl nonanoate.

Compositions of the present invention may comprise hydrogen peroxide and an organic hair follicle penetrating agent selected from ,hexyl methanoate, pentyl ethanoate, butyl propanoate, propyl butanoate, ethyl pentanoate, methyl hexanoate, heptyl methanoate, hexyl ethanoate, pentyl propanoate, butyl butanoate, propyl pentanoate, ethyl hexanoate, methyl heptanoate, octyl methanoate, heptyl ethanoate, hexyl propanoate, pentyl butanoate, butyl pentanoate, propyl hexanoate, ethyl heptanoate, methyl octanoate, nonyl methanoate, octyl ethanoate, heptyl propanoate, hexyl butanoate, pentyl pentanoate, butyl hexanoate, propyl heptanoate, ethyl octanoate, methyl nonanoate, decyl methanoate, nonyl ethanoate, octyl propanoate, heptyl butanoate, hexyl pentanoate, pentyl hexanoate, butyl heptanoate, propyl octanoate, ethyl nonanoate, methyl decanoate, undecyl methanoate, decyl ethanoate, nonyl propanoate, octyl butanoate, heptyl pentanoate, hexyl hexanoate, pentyl heptanoate, butyl octanoate, propyl nonanoate, ethyl decanoate, methyl undecanoate, dodecyl methanoate, undecyl ethanoate, decyl propanoate, nonyl butanoate, octyl pentanoate, heptyl hexanoate, hexyl heptanoate, pentyl octanoate, butyl nonanoate, propyl decanoate, ethyl undecanoate, methyl dodecanoate, tridecyl methanoate, dodecyl ethanoate, undecyl propanoate, decyl butanoate, nonyl pentanoate, octyl hexanoate, heptyl heptanoate, hexyl octanoate, pentyl nonanoate, butyl decanoate, propyl undecanoate, ethyl dodecanoate, methyl tridecanoate, tetradecyl methanoate, tridecyl ethanoate, dodecyl propanoate, undecyl butanoate, decyl pentanoate, nonyl hexanoate, octyl heptanoate, heptyl octanoate, hexyl nonanoate, pentyl decanoate, butyl undecanoate, propyl dodecanoate, ethyl tridecanoate, methyl tetradecanoate, pentadecyl methanoate, tetradecyl ethanoate, tridecyl propanoate, dodecyl butanoate, undecyl pentanoate, decyl hexanoate, nonyl heptanoate, octyl octanoate, heptyl nonanoate, hexyl decanoate, pentyl undecanoate, butyl dodecanoate; propyl tridecanoate, ethyl tetradecanoate, or methyl pentadecanoate.

Compositions of the present invention may comprise trichloroisocyanuric acid or sodium trichloroisocyanurate and an organic hair follicle penetrating agent selected from hexyl methanoate, pentyl ethanoate, butyl propanoate, propyl butanoate, ethyl pentanoate, methyl hexanoate, heptyl methanoate, hexyl ethanoate, pentyl propanoate, butyl butanoate, propyl pentanoate, ethyl hexanoate, methyl heptanoate, octyl methanoate, heptyl ethanoate, hexyl propanoate, pentyl butanoate, butyl pentanoate, propyl hexanoate, ethyl heptanoate, methyl octanoate, nonyl methanoate, octyl ethanoate, heptyl propanoate, hexyl butanoate, pentyl pentanoate, butyl hexanoate, propyl heptanoate, ethyl octanoate, methyl nonanoate, decyl methanoate, nonyl ethanoate, octyl propanoate, heptyl butanoate, hexyl pentanoate, pentyl hexanoate, butyl heptanoate, propyl octanoate, ethyl nonanoate, methyl decanoate, undecyl methanoate, decyl ethanoate, nonyl propanoate, octyl butanoate, heptyl pentanoate, hexyl hexanoate, pentyl heptanoate, butyl octanoate, propyl nonanoate, ethyl decanoate, methyl undecanoate, dodecyl methanoate, undecyl ethanoate, decyl propanoate, nonyl butanoate, octyl pentanoate, heptyl hexanoate, hexyl heptanoate, pentyl octanoate, butyl nonanoate, propyl decanoate, ethyl undecanoate, methyl dodecanoate, tridecyl methanoate, dodecyl ethanoate, undecyl propanoate, decyl butanoate, nonyl pentanoate, octyl hexanoate, heptyl heptanoate, hexyl octanoate, pentyl nonanoate, butyl decanoate, propyl undecanoate, ethyl dodecanoate, methyl tridecanoate, tetradecyl methanoate, tridecyl ethanoate, dodecyl propanoate, undecyl butanoate, decyl pentanoate, nonyl hexanoate, octyl heptanoate, heptyl octanoate, hexyl nonanoate, pentyl decanoate, butyl undecanoate, propyl dodecanoate, ethyl tridecanoate, methyl tetradecanoate, pentadecyl methanoate, tetradecyl ethanoate, tridecyl propanoate, dodecyl butanoate, undecyl pentanoate, decyl hexanoate, nonyl heptanoate, octyl octanoate, heptyl nonanoate, hexyl decanoate, pentyl undecanoate, butyl dodecanoate; propyl tridecanoate, ethyl tetradecanoate, or methyl pentadecanoate.

It will be appreciated that the amount of the organic hair follicle penetrating agent may vary depending on the physical and chemical properties of the oxidising agent contemplated for the composition (e.g. peroxide based or chlorine based oxidising agents), as well as on the chemical and physical properties of any other ingredients in the composition. The amount of organic hair follicle penetrating agent can be readily determined by routine experiment.

The ratio between the amount of oxidising agent and organic hair follicle penetrating agent in the topical composition according to the present invention may also vary depending on the activity and the physical and chemical properties of the oxidising agent, as well as on the properties of any other ingredients (*e.g*. solvents, stabilizers, preservatives, colouring agents, buffering agents *etc*.).

In certain embodiments of the present invention, the topical composition may include an amount of oxidising agent and an amount of an organic hair follicle penetrating agent that is in a ratio from 1:5 to 1:30, preferably from 1:10 to 1:30 and most preferably from 1:15 to 1:25.

When the composition comprises a peroxide based oxidising agent, the organic hair follicle penetrating agent may be present in an amount from 50% to 70% by weight, and most preferably from 60% to 70% by weight (*e.g*. approximately 65% by weight).

In embodiments where the peroxide based oxidising agent is hydrogen peroxide, the organic hair follicle penetrating agent may be present in an amount from 50% to 80%, preferably from 50% to 70%, more preferably from 60% to 70%. In the most preferred embodiment, the follicle penetrating agent is present in an amount of approximately 65%.

When the composition comprises a chlorine based oxidising agent, the organic hair follicle penetrating agent is present in an amount from from 50% to 99% by weight, more preferably from 70% to 99% by weight, more preferably from 80% to 99% by weight, even more preferably from 90% to 99% by weight, and most preferably from 93% to 96% by weight (*e.g*. 94% and 95% by weight).

In embodiments wherein the chlorine based oxidising agent is trichloroisocyanuric acid or sodium trichloroisocyanurate, the organic hair follicle penetrating agent is present in an amount from 50% to 99%, preferably from 80% to 99%, more preferably from 90% to 99%. In the most preferred embodiment, the follicle penetrating agent is present in an amount from approximately 94% to 95%.

In another specific preferred embodiment, the composition comprises hydrogen peroxide in an amount of approximately 3% by weight of the composition, butyl butanoate in an amount of approximately 65% by weight, ethanol in an amount of approximately 28% by weight, and water in an amount of approximately 5% by weight.

In a further specific preferred embodiment, the composition comprises hydrogen peroxide in an amount of approximately 3% by weight of the composition, hexyl hexanoate in an amount of approximately 65% by weight, ethanol in an amount of approximately 28% by weight, and water in an amount of approximately 5% by weight.

In yet another specific preferred embodiment, the composition comprises trichloroisocyanuric acid in an amount of approximately 5% by weight of the composition and butyl butanoate in an amount of approximately 94% to 95% by weight.

In a still further specific preferred embodiment, the composition comprises trichloroisocyanuric acid in an amount of approximately 5% by weight of the composition, hexyl hexanoate in an amount of approximately 85% to 86% by weight and acetone in an amount of approximately 9-10%.

In preferred embodiments of the present invention, the composition should be homogeneous (*e.g*. in the form of a solution) so that the oxidising agent may reach its intended site of action within the hair follicle. Certain contemplated combinations of oxidising agent and organic hair follicle penetrating agent may not form a homogeneous solution when mixed. In such situations, a solvent (or co-solvent if both the oxidising agent penetrating agent are liquids) may be used to solubilise the oxidising agent and penetrating agent. The amount of solvent needed to form a homogeneous solution will be readily determined by routine experimentation.

A variety of acceptable solvents are useful in the present invention. The solvents should be suitable for topical application to the skin and should not induce unacceptable levels of irritation on application to the skin, ideally even when the composition is applied as a "leave-on" treatment. The solvent may also appear non-greasy after topical application of the composition.

Typical suitable solvents that may be used include dipropylene glycol monomethyl ether, *N*,*N*-dimethylacetamide, propylene carbonate, propylene glycol, diethylene glycol monobutyl ether, glycerin triethanoate, triethyl citrate, acetyltriethyl citrate, acetyltributyl citrate, diethylene glycol monomethyl ether, diethylene glycol monoethyl ether, benzyl benzoate, ethylene glycol monobutyl ether, ethyl lactate, ethylene glycol monobutyl ether ethanoate, isopropanol, propanol, ethanol, benzyl alcohol, ethyl acetoacetate, 2-pyrrolidinone, dimethyl isosorbide, diacetone alcohol, tetrahydrofurfuryl alcohol, propylene glycol monomethyl ether, ethanol, diethyl phthalate, heptyl ethanoate, methyl caprylate/caprate, *N*,*N*-dimethylcaprylamide, pentyl ethanoate, hexyl ethanoate, cyclohexyl ethanoate, ethylene glycol diacetate, methoxypropyl ethanoate, furfuryl alcohol, dibutyl phthalate, *N*-methyl pyrrolidinone, glycerol formal, methyl salicylate, cinnamaldehyde, dimethyl sulfoxide, and cyclohexanone and mixtures thereof. Preferably, the solvent is ethanol, propanol or isopropanol or a mixture thereof, with ethanol being the most preferable.

The solvent may also be a C₃ to C₆ ester or ketone, or a mixture thereof.

Typical suitable C₃ to C₆ esters that may be used include, but are not limited to, methyl acetate, ethyl acetate, methyl propionate, methyl methoxyacetate, ethyl glycolate, propyl formate, isopropyl formate, butyl formate, *tert*-butyl formate, isobutyl formate, isopropyl acetate, ethyl propionate, methyl butyrate, methyl isobutyrate, dimethyl malonate, methyl acetoacetate, propyl acetate, methyl acetopyruvate, methyl 3-methoxypropionate, methyl 2-hydroxyisobutyrate, methyl levulinate, methyl 2-hydroxy-2-methyl-3-oxobutyrate, methyl 4-methoxyacetoacetate, methyl 2,2-dimethyl-3-hydroxypropionate dimethyl methylmalonate, ethylene glycol diacetate, ethyl methoxyacetate, ethyl acetoacetate, butyl acetate, sec-butyl acetate, *tert*-butyl acetate, ethyl butyrate, ethyl ethoxyacetate, ethyl isobutyrate, ethyl 3-hydroxybutyrate, 2-ethoxyethyl acetate, propyl 2-hydroxypropanoate, isopropyl propionate, isobutyl acetate, butyl glycolate, isopentyl formate, methyl 2-methybutyrate, methyl pivalate, methyl valerate, methyl isovalerate, methyl 4-methoxybutyrate or propyl propionate.

Typical suitable C₃ to C₆ ketones that may be used include, but are not limited to, acetone, hydroxyacetone, methoxyacetone, acetylacetone, cyclopropyl methyl ketone, 2-butanone, 3-hydroxy-2-butanone, 4-hydroxy-2-butanone, cyclobutanone, cyclobutyl methyl ketone, butanedione, 4-methoxybutan-2-one, methoxy-2-propanone, 2-pentanone, 3-pentanone, cyclopentanone, 3-methylcyclopentanone, 1,3-cyclopentanedione, 2-methyl-1,3-cyclopentanedione, 3-methyl-1,2-cyclopentanedione, 3-methyl-2,4-pentanedione, 3-methyl-2-butanone, 3-hydroxy-3-methyl-2-butanone, methyl acetopyruvate, 5-hydroxy-2-pentanone, 4-ethoxy-2-butanone, 4-hydroxy-4-methyl-2-pentanone, 2,3-pentanedione, 2-hexanone, 3-hexanone, 2,5-hexanedione, 3,4-hexanedione, 2,3-hexanedione, 4-hydroxy-3-hexanone, 2-methyl-3-pentanone, 3-methyl-2-pentanone, 4-methyl-2-pentanone. 3,3-dimethyl-2-butanone, cyclohexanone, 1,2-cyclohexanedione, 1,3-cyclohexanedione, or 1,4-cyclohexanedione.

When the composition comprises a peroxide based oxidising agent, any type of solvent may be used. However, when the composition is a chlorine based oxidising agent, the solvent is preferably an aprotic solvent, such as a C₃ to C₆ ester or ketone, or a mixture thereof.

When required, the solvent may be present in an amount from 0.1% to 50% by weight of the composition, preferably from 5% to 50% by weight, more preferably from 10% to 40% by weight, more preferably from 20% to 40% by weight and most preferably from 25% to 35% by weight (*e.g*. 28%).

When the composition comprises a chlorine based oxidising agent and the solvent is a C₃ to C₆ ester or ketone, or a mixture thereof, the solvent is present in an amount from 0.1% to 45% by weight of the composition, preferably from 0.1% to 25%, more preferably from 5% to 20% by weight, and most preferably from 5% to 15% by weight.

In general, aqueous solutions of hydrogen peroxide are not miscible with the organic hair follicle penetrating agents described herein, in particular butyl butanoate, due to the hydrophilicity of the hydrogen peroxide. In embodiments wherein the composition comprises hydrogen peroxide, a cosolvent, such as ethanol, may be present in an amount from 1% to 50% by weight of the composition, preferably from 10% to 40% by weight, more preferably from 20% to 40% by weight, even more preferably from 25% to 35% by weight, and most preferably from 27% to 29% by weight (*e.g*. approximately 28%).

A composition according to the present invention may contain, in addition to the oxidising agent and hair follicle penetrating agent and/or a solvent, one or more additional (suitably liquid) excipients. The excipients may include a stabilizer, a preservative, a colouring agent, an anti-inflammatory agent, water, a buffering agent, a thickening agent, an emollient, a perfuming agent or a mixture thereof. Any such excipient should ideally be acceptable for cosmetic use and, in particular, acceptable for topical administration to human skin.

Stabilizers may include, but are not limited to, glycol stearate or PEG-150 distearate. The stabilizer, when used, is typically present in an amount from about 0.1% to 5% by weight of the composition.

Preservatives may include, but are not limited to, tetrasodium ethylene-diamine tetraacetic acid (EDTA), methylparaben, benzophenone-4, methylchloroisothiazolinone, methylisothiazolinone or a mixture thereof. Preservatives, when used, are typically present in an amount from about 0.01% to 6% by weight of the composition, preferably about 0.05% to 4% by weight, and more preferably from about 0.1% to 2% by weight.

Anti-inflammatory agents may include non-steroidal anti-inflammatory agents such as ibuprofen, ketoprofen, flurbiprofen, diclofenac, naproxen.

Colouring agents may include, but are not limited to, FD&C Green No. 3, Ext. D&C Violet No. 2, FD&C Yellow No. 5, FD&C Red No. 40, and mixtures thereof. The coloring agents, when used, are typically present in an amount from about 0.001% to 0.1% by weight of the composition, and preferably from about 0.005% to 0.05% by weight.

Thickening agents may include cellulose-based thickening agents such as ethyl cellulose, hydroxyethyl cellulose, hydroxypropyl cellulose, hydroxypropyl methyl cellulose and carboxymethyl celluloses. Such agents may be used in the form of a (preferably pharmaceutically acceptable) salt such as for instance the sodium salt. A thickening agent may be a polymeric thickening agent such as a carbomer, which will typically be a homopolymer of acrylic acid, cross-linked with an allyl ether. The thickening agents, when used, are typically present in an amount from 0.2% to 2.5% by weight of the composition, preferably from 0.5% to 2.5% by weight, more preferably from 1.0% to 2.5%, and especially from 2.0% to 2.5% by weight.

Emollients may include mineral oil, petrolatum, silicone, silicone-glycol copolymers, triglyceride esters, acetylated monoglycerides, alkyl esters of fatty acids, fatty acids and alcohols, lanolin and lanolin derivatives, beeswax derivatives, polyhydric alcohol, and amides of fatty acids. The amount of emollient in the composition may range from 0.10 to 10% by weight of the composition, preferably from 0.5 to 7.0% by weight.

Humectants may also be added to the composition of the invention. Such humectants aid in the rehydration and maintenance of hydration of the treated area of skin. In general, a humectant aids in increasing the effectiveness of an emollient; it may reduce scaling, stimulate removal of built-up scale, moisturize and improve skin feel. Examples of suitable humectants are glycerin, propylene glycol, butylene glycol, diglycerol, or ester derivatives thereof, and sorbitol.

The composition may be in the form of a fluid, for example a lotion, cream, ointment, varnish, paste, gel or other viscous or semi-viscous fluid, or a less viscous fluid such as might be used in sprays, foams, drops or aerosols. The composition may in particular be in the form of a solution, lotion or gel. The composition may be applied to a carrier such as a sponge, swab, brush, pad, tissue, cloth, wipe, skin patch, dressing (or other material designed for application to the skin), to facilitate its topical administration. In preferred embodiments, the composition is in the form of a lotion on a carrier.

The composition of the present invention may be applied to any area of skin on the body, such as skin located on the chest, back, shoulders, abdomen, arm, underarm, hands, legs, feet, toes, bikini line, genital region, face, chin, upper lip, and eyebrows, or a combination thereof.

Ideally, the follicle penetrating agent should penetrate the vacant hair follicles but not penetrate the skin layer and dissolve the fatty acids on the surface of the skin and wet the skin surface itself, thus allowing penetration into the hair follicle and delivery of suitable compounds to the hair bulb and cells of the dermal papilla.

Another aspect of the present invention provides methods for removing hair and inhibiting its re-growth by topically applying a composition according to the present invention to vacant hair follicles. When used herein, the term *"removing hair"* refers to the removal of hair either in one or several treatments of the method disclosed herein. The method for removing hair and inhibiting its re-growth comprises:
(a) removing hair from an area of skin by epilation;
(b) topically applying a composition according to the present invention onto the area of skin having vacant hair follicles for a period of time sufficient to inhibit the re-growth of hair from the vacant follicles.

Any epilation technique that is known in the art may be used, such as plucking, waxing, sugaring, or tweezing.

The composition is applied to an area of skin which is to be treated prior to epilation, that is, step (b) of the method of the invention for removing hair and inhibiting its re-growth may be performed prior to step (a). In other words, the composition is applied on an area of skin having hair. Preferably, the composition should be applied to the area of skin one to ten minutes prior to epilation. Hair is then removed from the area of skin, which has been treated with the composition, by epilation. Without wishing to be bound by theory, it is believed that the removal of the hair from the follicle facilitates entry of the composition into the now vacant hair follicle by suction. Epilation is preferably performed by plucking or tweezing in order to minimise the removal of the composition during epilation. However, if a substantial amount of the composition is removed during the epilation process, another application of the composition may take place after epilation.

The composition may be applied as a "leave-on" treatment although it may be desirable to remove the composition in certain situations, such as if the human subject suffers from sensitive skin.

In embodiments of the present invention, the composition is applied to, or left on, an area of skin for a sufficient amount of time to inhibit re-growth of hair from the vacant follicles. When used in the context of the present application, a *"sufficient amount of time"* refers to the length of time it would take for a composition to penetrate a hair follicle and deliver an oxidising agent to the hair bulb and dermal papilla and for the oxidising agent to irreversibly damage the cells of the dermal papilla to such an extent that production of a viable hair is no longer possible.

In general, the time sufficient to inhibit hair re-growth is from one to ten minutes, and therefore it is preferable to remove the composition from one to ten minutes after application. More preferably the composition is removed from one to five minutes after application. Ideally, the skin should not be exposed to the composition for periods of more than 10 minutes. However, the skilled person understands that application time will depend upon the subject being treated and therefore exposure times of greater than 10 minutes may be necessary in some cases. The composition may be removed from the skin by any known method, for example, by being dabbed off or cleaned off with a tissue.

Furthermore, it is to be understood that the amount of the composition to be applied on to the skin will vary depending upon the size of the area being treated and the density of hair follicles in that particular region. For example, an area covered by 100 hairs will require more solution than an area covered by 10 hairs.

It is to be appreciated that patients requiring permanent hair removal may not respond to the method of the present invention in a consistent manner. It may therefore be necessary to repeat the hair removal method if any re-growth of hair from a treated area is observed. In such situations it is intended that the method should be repeated as many times as is necessary to inhibit hair re-growth to a level that is acceptable to the patient.

An aspect of the present invention provides for a method for preparing a composition according to the invention, wherein the method involves mixing the oxidising agent and the organic hair follicle penetrating agent, optionally with a solvent and/or one or more additional excipients, wherein the concentration of the oxidising agent in the resultant composition is between 0.1% and 10% by weight of the composition. The ingredients of the composition may be mixed together in any conventional manner. In one embodiment, the ingredients of the composition are added to a vessel and mixed together, preferably at a temperature between 10 °C and 25 °C. Whilst the order in which most ingredients are added to the vessel has no bearing on the properties of the resultant composition, preferably the oxidising agent should be the final ingredient added to the mixture. Preferably the resultant mixture is in the form of a homogeneous solution and therefore the method may further comprise a filtration step to remove any insoluble particulate matter from the composition. Alternatively, if the oxidising agent and the organic hair follicle penetrating agent are both liquids and the mixture thereof is not homogeneous (*e.g*.. it is an emulsion), a co-solvent should be added to the mixture in an amount necessary to obtain homogeneity. The final concentration of oxidising agent in the composition may be measured by any standard analytical procedure. For instance, the potassium permanganate titration for peroxide based oxidising agents (*e.g*. hydrogen peroxide) or the DPD (*N*,*N*-diethyl-*p*-phenylenediamine) test for chlorine based oxidising agents.

Another aspect of the present invention provides a composition according to the invention for use in the treatment of a condition characterised by excessive undesired hair growth. Conditions characterised by excessive undesired hair growth include congenital hypertrichosis, acquired hypertrichosis and hirsutism. In an embodiment of this aspect the composition is used to inhibit re-growth of hair in a vacant hair follicle by destroying and permanently inactivating the dermal papilla.

A further aspect of the present invention provides the use of a composition according to the invention, for non-therapeutic (e.g. cosmetic) purposes. In an embodiment of this aspect, the composition is used to inhibit re-growth of hair in a vacant hair follicle by destroying and permanently inactivating the dermal papilla, for example, for cosmetic purposes such as to improve skin aesthetics.

In another aspect of the present invention, there is provided a kit, wherein the kit includes a first container containing an oxidising agent, as described herein; and a second container containing an organic hair follicle penetrating agent as described herein.

In embodiments where the oxidising agent and an organic hair follicle penetrating agent which are not readily miscible, the kit may optionally include a suitable solvent, as described herein, in a separate container or in combination with the oxidising agent and/or the organic hair follicle penetrating agent.

In another aspect of the present invention, there is provided a kit including a container having a composition according to the present invention, as herein described.

The kits described herein may further include a device to topically administer the composition according to the present invention to the site of administration. Such devices include a sponge, swab, brush, pad, tissue, cloth, wipe, skin patch, dressing (or other material designed for application of a topical composition to the skin).

The kits according to the present invention may further include written instructions as to the use of the components in preventing the re-growth of hair from vacant hair follicles. For instance, when the kit includes a first container containing an oxidising agent as described above; and a second container containing an organic hair follicle penetrating agent as described above, the instructions may provide directions for mixing the contents of the first and second containers prior to application or for the sequential application of the contents of the first and second containers. Methods of application may also be described. The instructions may also specify the length of time that the components of the kit should be applied to an area of skin, either sequentially or together, and/or provide instructions for removal of the components or the resultant composition from the application area. The time should be a sufficient length of time as defined above. Alternatively, when the kit includes a container having a composition according to the present invention, the instructions may provide directions for the application of the composition. The instructions may also specify the length of time that the composition should be applied to an area of skin and/or provide instructions for removal of the composition from the application area. This should be a sufficient length of time as defined above.

Having described the invention with reference to certain preferred embodiments, other embodiments will become apparent to one skilled in the art from consideration of the specification. The invention is further illustrated by reference to the following examples describing in detail the formation of the topical composition and methods of using the composition for the permanent removal of hair. It will be apparent to those skilled in the art that many modifications, both to materials and methods, may be practised without departing from the scope of the invention. The following experiments involved preparing topical compositions according to the present invention, and testing their ability to prevent the re-growth of hair from vacant follicles.

### Procedures for preparing compositions according to the present invention

### Example 1 (Reference Example)

Preparation of a composition containing an organic hair follicle penetrating agent (propyl propanoate) and a peroxide based oxidising agent (hydrogen peroxide).

### Materials

Propyl propanoate ≥98%, FCC, FG (Sigma Aldrich)
Food grade hydrogen peroxide (35% wt. in H₂O)
Ethanol ≥99.5%

### Method

A 35% by weight aqueous solution of hydrogen peroxide (7.08 mL, 8.0 g) was added to a stirred solution of propyl propanoate (81.6 mL, 68.0 g) and ethanol (35.5 mL, 28.0 g). The mixture was stirred at room temperature until a homogeneous solution was obtained.

The resulting solution had the following composition:
- propyl propanoate (approx. 68% by weight)
- ethanol (approx. 28% by weight)
- water (approx. 5.2% by weight)
- hydrogen peroxide (approx. 2.8% by weight)

The peroxide content was tested using the volumetric MnO₂ decomposition method. For example, see chemistry.slss.ie/resources/downloads/ch_me_6.1student.doc.

### Example 2

Preparation of a composition containing an organic hair follicle penetrating agent (butyl butanoate) and a chlorine based oxidising agent (trichloroisocyanuric acid).

### Materials

Butyl butanoate ≥98%, FCC, FG (Sigma Aldrich)
Trichloroisocyanuric acid NSF Standard 60

### Method

Trichloroisocyanuric acid (5.0 g) was added to a stirred solution of ≥98% butyl butanoate (109.3 mL, 95.0 g) and the resulting mixture was stirred at room temperature for 10 minutes. After this time the solution was filtered to remove any particulate matter.

The resulting solution had the following composition:
- butyl butanoate (approx. 95% by weight)
- trichloroisocyanuric acid (approx. 5% by weight)

The chlorine content was tested using an iodometric back titration method. For example, see www.hach.com/cms/documents/pdf/LIT/L7019-ChlorineAnalysis.pdf.

### Example 3

Preparation of a composition containing an organic hair follicle penetrating agent (hexyl hexanoate) and a chlorine based oxidising agent (trichloroisocyanuric acid).

### Materials

Hexyl hexanoate ≥98%, FCC, FG (Sigma Aldrich)
Trichloroisocyanuric acid NSF Standard 60
Acetone ACS reagent, ≥99.5% (Sigma Aldrich)

### Method

Trichloroisocyanuric acid (5.0 g) was added to a stirred solution of ≥98% hexyl hexanoate (100.0 mL, 86.2 g) and acetone (11.1 mL, 8.8 g) and the resulting mixture was stirred at room temperature for 10 minutes. After this time the solution was filtered to remove any particulate matter.

The resulting solution had the following composition:
- hexyl hexanoate (approx. 86% by weight)
- acetone (approx. 9% by weight)
- trichloroisocyanuric acid (approx. 5% by weight)

The chlorine content was tested using an iodometric back titration method. For example, see www.hach.com/cms/documents/pdf/LIT/L7019-ChlorineAnalysis.pdf.

### Comparative Example 1

Preparation of a composition containing an organic hair follicle penetrating agent (heptanol) and an inhibitor of cellular respiration (atractyloside).

### Materials

Heptanol >99.5%, GC (Sigma Aldrich)
Atractytyloside sodium salt (Sigma Aldrich)

### Method

Atractyloside sodium salt (1 mg) was added to a stirred solution of ≥99.5% heptanol (1 mL, 0.8 g) and the resulting mixture was stirred at room temperature for 10 minutes until a homogeneous solution was obtained.

### Hair re-growth studies

### Materials

The following compositions were tested for their ability to prevent the re-growth of hair from vacant hair follicles:
- the composition of Example 1
- the composition of Comparative Example 1

### General Procedure

Hair (50 hairs) was removed by epilation from a 2 cm² area of skin (armpit). After two hours the topical composition was applied to this area of skin in the form of a lotion on a carrier strip. The composition was left on the skin for three minutes, after which time the composition was removed by dabbing off. Three months after the composition had been applied, the treated area was analysed to determine the number of hairs that had re-grown

### Results

After 3 months, 33 hairs were counted in the area of skin treated with the composition of Example 1. This equates to a 35% reduction in the number of hairs present.

After 3 months, 42 hairs were counted in the area of skin treated with the composition of comparative Example 1. This equates to a 15% reduction in the number of hairs present.

## Claims

1. A composition for inhibiting the re-growth of hair from a vacant hair follicle comprising at least one organic hair follicle penetrating agent and at least one oxidising agent, **characterised in that** the at least one hair follicle penetrating agent is a C₇ to C₁₆ ester or a mixture thereof and that the total weight of the hair follicle penetrating agent is from 50% to 99% by weight of the composition, wherein the at least one oxidising agent is selected from a peroxide based oxidising agent or a chlorine based oxidising agent, and wherein the peroxide based oxidising agent is hydrogen peroxide, *tert*-butyl hydroperoxide, benzoyl peroxide, cumene hydroperoxide, peroxydisulfuric acid or a salt thereof, peroxyphosphoric acid or a salt thereof, peroxyacetic acid or a salt thereof, peroxypropionic acid or a salt thereof, or peroxybutyric acid or a salt thereof.

2. A composition according to Claim 1, wherein the chlorine based oxidising agent is sodium hypochlorite, calcium hypochlorite, trichloroisocyanuric acid or a salt thereof, dichloroisocyanuric acid or a salt thereof, monochloroisocyanuric acid or a salt thereof, an inorganic chloramine, chloramine T, or halozone wherein the chlorine based oxidising agent is preferably trichloroisocyanuric acid or a salt thereof.

3. A composition according to Claim 1, wherein the peroxide based oxidising agent is hydrogen peroxide.

4. A composition according to any one of Claims 1 to 3, wherein the at least one organic hair follicle penetrating agent is:
a C₈ ester selected from heptyl methanoate, hexyl ethanoate, pentyl propanoate, butyl butanoate, propyl pentanoate, ethyl hexanoate and methyl heptanoate, wherein the C₈ ester is preferably butyl butanoate; or
a C₁₂ ester selected from undecyl methanoate, decyl ethanoate, nonyl propanoate, octyl butanoate, heptyl pentanoate, hexyl hexanoate, pentyl heptanoate, butyl octanoate, propyl nonanoate, ethyl decanoate, and methyl undecanoate, wherein the C₁₂ ester is preferably hexyl hexanoate.

5. A composition according to any one of Claims 1 to 4, wherein the total weight of the oxidising agent is from 0.1% to 10% by weight of the composition.

6. A composition according to any one of Claims 1 to 5, further comprising a solvent wherein the solvent is optionally an a C₃ to C₆ ester or ketone, or a mixture thereof, or alcohol, and wherein the alcohol is preferably ethanol, propanol or isopropanol.

7. A composition according to Claim 6, wherein the total weight of the solvent is from 10% to 40% by weight of the composition.

8. A composition according to any one of Claim 1 and Claims 3 to 7, wherein the at least one oxidising agent is a peroxide based oxidising agent in an amount from 2% to 6% by weight of the composition, the at least one organic hair follicle penetrating agent is in an amount from 60% to 70% by weight of the composition, and the composition further comprises solvent in an amount from 25% to 35% by weight of the composition.

9. A composition according to any one of Claims 1, 2 and 4 to 7, wherein the at least one oxidising agent is a chlorine based oxidising agent in an amount from 1% to 15% by weight of the composition, and the at least one hair follicle penetrating agent is in an amount from 85% to 99% by weight of the composition.

10. A method for preparing a composition according to any one of Claims 1 to 9 comprising forming a mixture of the oxidising agent and the organic hair follicle penetrating agent, and optionally a solvent and/or one or more additional excipients, wherein the concentration of the oxidising agent in the resultant composition is between 0.1% and 10% by weight of the composition.

11. A method for removing hair and inhibiting its re-growth comprising the steps of:
a. removing hair from an area of skin by epilation;
b. topically applying a composition for inhibiting the re-growth of hair from a vacant hair follicle onto the area of skin for a period of time sufficient to inhibit the re-growth of hair from the vacant follicles, wherein the composition is applied to the area of skin prior to epilation, and wherein the composition is a composition according to any one of Claims 1 to 9.

12. Use of a composition according to any one of Claims 1 to 9 to inhibit the re-growth of hair.

13. A composition according to any one of Claims 1 to 9 for use in the treatment of a condition **characterised by** excessive undesired hair growth, wherein the condition is optionally congenital hypertrichosis, acquired hypertrichosis or hirsutism.

14. A kit comprising a first container that contains an oxidising agent according to any one of Claims 1 to 3 and a second container that contains an organic hair follicle penetrating agent according to any one of Claims 1 and 4.

## Patentansprüche

1. Zusammensetzung zum Hemmen des Haarwuchses aus einem vakanten Haarfollikel, umfassend mindestens ein organisches Haarfollikel penetrierendes Mittel und mindestens ein oxidierendes Mittel, **dadurch gekennzeichnet, dass** das mindestens eine Haarfollikel penetrierende Mittel ein C₇ bis C₁₆-Ester oder eine Mischung davon ist und das Gesamtgewicht des Haarfollikel penetrierenden Mittels von 50% bis 99 Gew.-% der Zusammensetzung beträgt, wobei das mindestens eine oxidierende Mittel ausgewählt ist aus einem oxidierenden Mittel auf Basis von Peroxid oder einem oxidierenden Mittel auf Basis von Chlor, und wobei das auf Peroxid basierende Mittel Wasserstoffperoxid, *tert*-Butylhydroperoxid, Benzoylperoxid, Cumolhydroperoxid, Peroxydischwefelsäure oder ein Salz davon, Peroxyphosphorsäure oder ein Salz davon, Peroxyessigsäure oder ein Salz davon, Peroxypropionsäure oder ein Salz davon oder Peroxybuttersäure oder ein Salz davon ist.

2. Zusammensetzung nach Anspruch 1, wobei das auf Chlor basierende oxidierende Mittel Natriumhypochlorit, Calciumhypochlorit, Trichlorisocyanursäure oder ein Salz davon, Dichlorisocyanursäure oder ein Salz davon, Monochlorisocyanursäure oder ein Salz davon, ein anorganisches Chloramin, Chloramin T oder Halazon ist, wobei das auf Chlor basierende oxidierende Mittel bevorzugt Trichlorisocyanursäure oder ein Salz davon ist.

3. Zusammensetzung nach Anspruch 1, wobei das auf Peroxid basierende oxidierende Mittel Wasserstoffperoxid ist.

4. Zusammensetzung nach einem der Ansprüche 1 bis 3, wobei das mindestens eine organische Haarfollikel penetrierende Mittel:
ein C₈-Ester ist, der ausgewählt ist aus Heptylmethanoat, Hexylethanoat, Pentylpropanoat, Butylbutanoat, Propylpentanoat, Ethylhexanoat und Methylheptanoat, wobei der C₈-Ester bevorzugt Butylbutanoat ist; oder
ein C₁₂-Ester ist, der ausgewählt ist aus Undecylmethanoat, Decylethanoat, Nonylpropanoat, Octylbutanoat, Heptylpentanoat, Hexylhexanoat, Pentylheptanoat, Butyloctanoat, Propylnonanoat, Ethyldecanoat und Methylundecanoat, wobei der C₁₂-Ester bevorzugt Hexylhexanoat ist.

5. Zusammensetzung nach einem der Ansprüche 1 bis 4, wobei das Gesamtgewicht des oxidierenden Mittels von 0,1% bis 10 Gew.-% der Zusammensetzung beträgt.

6. Zusammensetzung nach einem der Ansprüche 1 bis 5, ferner umfassend ein Lösemittel, wobei das Lösemittel wahlweise ein C₃- bis C₆-Ester oder -Keton oder eine Mischung davon oder Alkohol ist, wobei der Alkohol bevorzugt Ethanol, Propanol oder Isopropanol ist.

7. Zusammensetzung nach Anspruch 6, wobei das Gesamtgewicht des Lösemittels von 10% bis 40 Gew.-% der Zusammensetzung beträgt.

8. Zusammensetzung nach einem der Ansprüche 1 und Ansprüche 3 bis 7, wobei das mindestens eine oxidierende Mittel ein auf Peroxid basierendes oxidierendes Mittel in einer Menge von 2% bis 6 Gew.-% der Zusammensetzung ist, das mindestens eine organische Haarfollikel penetrierende Mittel in einer Menge von 60% bis 70 Gew.-% der Zusammensetzung vorliegt, und die Zusammensetzung ferner Lösemittel in einer Menge von 25% bis 35 Gew.-% der Zusammensetzung umfasst.

9. Zusammensetzung nach einem der Ansprüche 1, 2 und 4 bis 7, wobei das mindestens eine oxidierende Mittel ein auf Chlor basierendes oxidierendes Mittel in einer Menge von 1% bis 15 Gew.-% der Zusammensetzung ist und das mindestens eine Haarfollikel penetrierende Mittel in einer Menge von 85% bis 99 Gew.-% der Zusammensetzung vorliegt.

10. Verfahren zum Herstellen einer Zusammensetzung nach einem der Ansprüche 1 bis 9, umfassend Bilden einer Mischung des oxidierenden Mittels und des organischen Haarfollikel penetrierenden Mittels und wahlweise eines Lösemittels und/oder eines oder mehrerer zusätzlicher Hilfsstoffe, wobei die Konzentration des oxidierenden Mittels in der resultierenden Zusammensetzung zwischen 0,1% und 10 Gew.-% der Zusammensetzung beträgt.

11. Verfahren zum Entfernen von Haar und zum Hemmen seines Nachwachsens, umfassend die Schritte:
a. Entfernen von Haar von einem Bereich der Haut durch Epilation;
b. topisches Auftragen einer Zusammensetzung zum Hemmen des Nachwachsens von Haar aus einem vakanten Haarfollikel auf dem Bereich der Haut für eine Zeitdauer, die ausreichend ist, um das Nachwachsen von Haar aus den vakanten Haarfollikeln zu hemmen, wobei die Zusammensetzung auf den Bereich der Haut vor der Epilation aufgetragen wird und wobei die Zusammensetzung eine Zusammensetzung nach einem der Ansprüche 1 bis 9 ist.

12. Verwendung einer Zusammensetzung nach einem der Ansprüche 1 bis 9 zum Hemmen des Nachwachsens von Haar.

13. Zusammensetzung nach einem der Ansprüche 1 bis 9 zur Verwendung in der Behandlung eines Zustands, der **gekennzeichnet ist durch** übermäßiges unerwünschtes Haarwachstum, wobei der Zustand wahlweise angeborene Hypertrichose, erworbene Hypertrichose oder Hirsutismus ist.

14. Kit, umfassend einen ersten Behälter, der ein oxidierendes Mittel nach einem der Ansprüche 1 bis 3 enthält, und einen zweiten Behälter, der ein organisches Haarfollikel penetrierendes Mittel nach einem der Ansprüche 1 bis 4 enthält.

## Revendications

1. Composition pour inhiber la repousse d'un poil à partir d'un follicule pileux vide, comprenant au moins un agent organique pénétrant les follicules pileux et au moins un agent oxydant, **caractérisée en ce que** ledit au moins un agent pénétrant les follicules pileux est un ester en C₇ à C₁₆ ou un mélange de ceux-ci et **en ce que** le poids total de l'agent pénétrant les follicules pileux est à 50% à 99% en poids de la composition, dans laquelle ledit au moins un agent oxydant est choisi parmi un agent oxydant à base de peroxyde ou un agent oxydant à base de chlore, et dans laquelle l'agent oxydant à base de peroxyde est le peroxyde d'hydrogène, l'hydroperoxyde de *tert*-butyle, le peroxyde de benzoyle, l'hydroperoxyde de cumène, l'acide peroxydisulfurique ou un sel de celui-ci, l'acide peroxyphosphorique ou un sel de celui-ci, l'acide peroxyacétique ou un sel de celui-ci, l'acide peroxypropionique ou un sel de celui-ci, ou l'acide peroxybutyrique ou un sel de celui-ci.

2. Composition selon la revendication 1, dans laquelle l'agent oxydant à base de chlore est l'hypochlorite de sodium, l'hypochlorite de calcium, l'acide trichloroisocyanurique ou un sel de celui-ci, l'acide dichloroisocyanurique ou un sel de celui-ci, l'acide monochloroisocyanurique ou un sel de celui-ci, une chloramine inorganique, la chloramine T, ou l'halozone, dans laquelle l'agent oxydant à base de chlore est de préférence l'acide trichloroisocyanurique ou un sel de celui-ci.

3. Composition selon la revendication 1, dans laquelle l'agent oxydant à base de peroxyde est le peroxyde d'hydrogène.

4. Composition selon l'une quelconque des revendications 1 à 3, dans laquelle ledit au moins un agent organique pénétrant les follicules pileux est:
un ester en C₈ choisi parmi le méthanoate d'heptyle, l'éthanoate d'hexyle, le propanoate de pentyle, le butanoate de butyle, le pentanoate de propyle, l'hexanoate d'éthyle et l'heptanoate de méthyle, lequel ester en C₈ étant de préférence le butanoate de butyle; ou
un ester en C₁₂ choisi parmi le méthanoate d'undécyle, l'éthanoate de décyle, le propanoate de nonyle, le butanoate d'octyle, le pentanoate d'heptyle, l'hexanoate d'hexyle, l'heptanoate de pentyle, l'octanoate de butyle, le nonanoate de propyle, le décanoate d'éthyle et l'undécanoate de méthyle, lequel ester en C₁₂ étant de préférence l'hexanoate d'hexyle.

5. Composition selon l'une quelconque des revendications 1 à 4, dans laquelle le poids total de l'agent oxydant est de 0,1% à 10% en poids de la composition.

6. Composition selon l'une quelconque des revendications 1 à 5, comprenant en outre un solvant, dans laquelle le solvant est facultativement un ester ou une cétone en C₃ à C₆, ou un mélange de ceux-ci, ou un alcool, et dans laquelle l'alcool est de préférence l'éthanol, le propanol ou l'isopropanol.

7. Composition selon la revendication 6, dans laquelle le poids total du solvant est de 10% à 40% en poids de la composition.

8. Composition selon l'une quelconque parmi la revendication 1 et les revendications 3 à 7, dans laquelle ledit au moins un agent oxydant est un agent oxydant à base de peroxyde en une quantité de 2% à 6% en poids de la composition, ledit au moins un agent organique pénétrant les follicules pileux est en une quantité de 60% à 70% en poids de la composition, et la composition comprend en outre un solvant en une quantité de 25% à 35% en poids de la composition.

9. Composition selon l'une quelconque des revendications 1, 2 et 4 à 7, dans laquelle ledit au moins un agent oxydant est un agent oxydant à base de chlore en une quantité de 1% à 15% en poids de la composition, et ledit au moins un agent pénétrant les follicules pileux est en une quantité de 85% à 99% en poids de la composition.

10. Procédé pour préparer une composition selon l'une quelconque des revendications 1 à 9, comprenant la formation d'un mélange de l'agent oxydant et de l'agent organique pénétrant les follicules pileux, et facultativement d'un solvant et/ou d'un ou plusieurs excipients supplémentaires, dans lequel la concentration de l'agent oxydant dans la composition obtenue est entre 0,1% et 10% en poids de la composition.

11. Procédé pour enlever des poils et inhiber leur repousse, comprenant les étapes consistant:
a. à enlever les poils d'une zone de la peau par épilation;
b. à appliquer topiquement une composition pour inhiber la repousse des poils à partir d'un follicule pileux vide sur la zone de la peau pendant une période de temps suffisante pour inhiber la repousse des poils à partir des follicules pileux vides, la composition étant appliquée à la zone de peau avant l'épilation, et la composition étant une composition selon l'une quelconque des revendications 1 à 9.

12. Utilisation d'une composition selon l'une quelconque des revendications 1 à 9 pour inhiber la repousse de poils.

13. Composition selon l'une quelconque des revendications 1 à 9, pour l'utilisation dans le traitement d'une affection **caractérisée par** une croissance pileuse excessive indésirable, dans laquelle l'affection est facultativement une hypertrichose congénitale, une hypertrichose acquise ou un hirsutisme.

14. Kit comprenant un premier récipient qui contient un agent oxydant selon l'une quelconque des revendications 1 à 3 et un second récipient qui contient un agent organique pénétrant les follicules pileux selon l'une quelconque des revendications 1 et 4.
